Europäisches Patentamt

European Patent Office

Office européen des brevets

(19)

(11) EP 0 778 019 A1

(12) EUROPÄISCHE PATENTANMELDUNG

(43) Veröffentlichungstag:
11.06.1997 Patentblatt 1997/24

(51) Int. Cl.$^6$: A61K 7/13

(21) Anmeldenummer: 96116147.8

(22) Anmeldetag: 09.10.1996

(84) Benannte Vertragsstaaten:
DE ES FR GB IT

(30) Priorität: 08.12.1995 DE 19545837

(71) Anmelder: Wella Aktiengesellschaft
64274 Darmstadt (DE)

(72) Erfinder: Balzer, Wolfgang R., Dr.
64274 Darmstadt (DE)

(54) **Oxidationshaarfärbemittel**

(57) Die Erfindung betrifft ein Mittel zum oxidativen Färben von Haaren, welches als Entwicklersubstanz eine Mischung aus 2-(2,5-Diaminophenyl)ethanol und 4-Amino-2-aminomethyl-phenol enthält.

EP 0 778 019 A1

**Beschreibung**

Gegenstand der Erfindung sind Mittel zur oxidativen Färbung von Haaren, dadurch gekennzeichnet, daß sie als Entwicklersubstanzen 2-(2,5-Diaminophenyl)ethanol und 4-Amino-2-aminomethyl-phenol in Kombination mit Kupplungssubstanzen enthalten.

Auf dem Gebiet der Haarfärbung haben Oxidationsfarbstoffe eine wesentliche Bedeutung erlangt. Die Färbung entsteht hierbei durch Reaktion bestimmter Entwicklersubstanzen mit bestimmten Kupplersubstanzen in Gegenwart eines geeigneten Oxidationsmittels. Als Entwicklersubstanzen werden, insbesondere 2,5-Diaminotoluol, 2,5-Diamino-phenylethyl-alkohol, p-Aminophenol und 1,4-Diaminobenzol eingesetzt. Von den vorzugsweise verwendeten Kupplersubstanzen sind Resorcin, 4-Chlorresorcin, 1-Naphthol, m-Aminophenol und Derivate des m-Phenylendiamins zu nennen. An Oxidationsfarbstoffe, die zur Färbung menschlicher Haare Verwendung finden, sind zahlreiche besondere Anforderungen gestellt. So müssen sie in toxikologischer und dermatologischer Hinsicht unbedenklich sein und Färbungen in der gewünschten Intensität ermöglichen. Ferner wird für die erzielten Haarfärbungen eine gute Licht-, Dauerwell-, Säure- und Reibechtheit gefordert. Auf jeden Fall aber müssen solche Haarfärbungen ohne Einwirkung von Licht, Reibung und chemischen Mitteln über einen Zeitraum von mindestens 4 bis 6 Wochen stabil bleiben. Außerdem ist es erforderlich, daß durch Kombination geeigneter Entwickler- und Kupplerkomponenten eine breite Palette verschiedener Farbnuancen erzeugt werden kann.

Die meisten der derzeit eingesetzten Kombinationen erfüllen zwar weitgehend die gestellten Anforderungen, zeigen jedoch in dem einen oder anderen Punkt noch Nachteile. Besonders nachteilig erweist sich hierbei, wenn durch nachfolgende Einflüsse, wie zum Beispiel Lichteinwirkung oder durch die Anwendung von Dauerwellbehandlungen, die erzielten Farbtöne verändert werden, so daß unbefriedigende Farbresultate resultieren.

So ermöglichen zwar Färbungen, die als Entwickler auf 4-Amino-2-aminomethyl-phenol beruhen, intensive Anfärbungen im Rotbereich. Durch Zusatz weiterer Entwickler wie zum Beispiel 2,5-Diamino-toluol läßt sich die Farbpalette auch auf natürliche Töne ausweiten, die allerdings die genannten Schwächen zeigen.

Es bestand daher die Aufgabe, geeignete Farbstoff-Kombinationen zur Verfügung zu stellen, die den erwähnten Anforderungen gerecht werden und gerade im Hinblick auf die Farbstabilität eine größere Konstanz zeigen.

Hierzu wurde nun völlig überraschend gefunden, daß durch ein Mittel zur oxidativen Färbung von Haaren mit einem Gehalt an einer Entwicklersubstanz-Kupplersubstanz-Kombination, welches als Entwicklungssubstanz ein Gemisch aus 2-(2,5-Diaminophenyl)-ethanol und 4-Amino-2-aminomethyl-phenol enthält, diese Aufgabe hervorragend gelöst wird.

Das erfindungsgemäße Haarfärbemittel enthält vorzugsweise nur 2-(2,5-Diaminophenyl)-ethanol und 4-Amino-2-aminomethyl-phenol als Entwicklersubstanz, jedoch können zur Erzielung bestimmter Farbtöne noch weitere Entwicklersubstanzen enthalten sein.

In dem erfindungsgemäßen Haarfärbemittel sollen die vorstehend genannten Entwicklungssubstanzen jeweils in einer für die Haarfärung ausreichenden Menge, im allgemeinen in einer Menge von etwa 0,01 bis 5,0 Gewichtsprozent, vorzugsweise 0,1 bis 3,5 Gewichtsprozent, enthalten sein.

Als Kupplersubstanzen sind für das hier beschriebene Haarfärbemittel, insbesondere Resorcin, 4-Chlorresorcin, 4,6-Dichlorresorcin, 2-Methylresorcin, 2,4-Diamino-5-fluortoluol, 2-Amino-4-(2'-hydroxyethylamino)-anisol, m-Phenylendiamin, 5-Amino-2-methylphenol, 2,4-Diaminophenoxyethanol, 1-Naphthol, m-Aminophenol, 3-Amino-4-chlor-6-methylphenol, 3-Amino-2-chlor-6-methyl-phenol, 3-Amino-4-fluor-6-methylphenol, 3-Amino-4-methoxy-6-methylphenol, 3-Amino-2-methylphenol, 4-Amino-2-hydroxy-phenoxyethanol, 4-Hydroxy-1,2-methylendioxybenzol, 4-(2'-Hydroxyethylamino)-1,2-methylendioxybenzol, 2,4-Diamino-5-ethoxytoluol, 4-Hydroxyindol und 3,5-Diamino-2,6-dimethoxypyridin geeignet, wobei eine für die Haarfärbung ausreichende Menge der einzelnen Kupplersubstanz im allgemeinen 0,01 bis 3,0 Gewichtsprozent, vorzugsweise 0,1 bis 2,5 Gewichtsprozent, eingesetzt wird.

Besonders bevorzugt sind hierbei Resorcin, 2-Methylresorcin, 5-Amino-2-methyl-phenol, 3-Amino-4-fluor-6-methylphenol, 4-(2'-Hydroxyethylamino)-1,2-methylendioxy-benzol, 2,4-Diamino-5-fluor-toluol, 2-Amino-4-(2'-hydroxyethylamino)-anisol und 1-Naphthol in den angegebenen Konzentrationen.

Die Gesamtmenge der in dem hier beschriebenen Haarfärbemittel enthaltenen Entwicklersubstanz-Kupplersubstanz-Kombination soll etwa 0,02 bis 8,0 Gewichtsprozent, vorzugsweise 0,2 bis 6,0 Gewichtsprozent, betragen. Die Entwicklersubstanzen werden im allgemeinen in etwa äquimolaren Mengen, bezogen auf die Kupplersubstanzen, eingesetzt. Es ist jedoch nicht nachteilig, wenn die Entwicklersubstanz in einem gewissen Überschuß oder Unterschuß vorhanden ist. Weiterhin kann das Haarfärbemittel dieser Anmeldung zusätzlich andere Farbkomponenten, beispielsweise 6-Amino-2-methylphenol und 2-Amino-5-methylphenol sowie ferner übliche direktziehende Farbstoffe, zum Beispiel Triphenylmethanfarbstoffe wie Basic Violet 14 (C. I. 42 510) und Basic Violet 2 (C. I. 42 520), aromatische Nitrofarbstoffe wie 2-Amino-4,6-dinitrophenol, 2-Amino-5-(2'-hydroxyethylamino)-nitrobenzol, 4-(2'Hydroxyethylamino)-3-nitrotoluol und 1-[(2'-Ureidoethyl)amino]-4-nitrobenzol und Azofarbstoffe wie Acid Brown 4 (C. I. 14 805) und Dispersionsfarbstoffe wie beispielsweise 1,4-Diaminoanthrachinon und 1,4,5,8-Tetraaminoanthrachinon, enthalten, wobei die Einsatzmenge etwa 0,1 bis 4,0 Gewichtsprozent beträgt. Selbstverständlich können die Kuppler- und Entwicklersubstanzen sowie die anderen Farbkomponenten, sofern es Basen sind, auch in Form der physiologisch verträglichen Säureadditionssalze wie beispielsweise als Hydrochlorid beziehungsweise Sulfat oder - sofern sie aromatische OH-Gruppen besitzen - in Form der Salze mit Basen, zum Beispiel als Alkaliphenolate, eingesetzt werden.

Darüber hinaus können dem Haarfärbemittel noch weitere übliche kosmetische Zusätze, beispielsweise Antioxidantien wie Ascorbinsäure, Thioglykolsäure oder Natriumsulfit, Parfümöle, Komplexbildner, Netzmittel, Emulgatoren, Verdicker und Pflegestoffe, zugesetzt werden. Die Zubereitungsform kann beispielsweise eine Lösung, insbesondere eine wäßrige oder wäßrig-alkoholische Lösung sein. Die besonders bevorzugten Zubereitungsformen sind jedoch eine Creme, ein Gel oder eine Emulsion. Ihre Zusammensetzung stellt eine Mischung der Farbstoffkomponenten in den für solche Zubereitungen üblichen Zusätzen dar. Übliche Zusätze in Lösungen, Cremes, Emulsionen und Gelen sind zum Beispiel Lösungsmittel wie Wasser, niedere aliphatische Alkohole, beispielsweise Ethanol, Propanol und Isopropanol oder Glykole wie 1,2-Propylenglykol, Glycerin, weiterhin Netzmittel oder Emulgatoren aus den Klassen der anionischen, kationischen, amphoteren oder nichtionogenen oberflächenaktiven Substanzen wie zum Beispiel Fettalkoholsulfate, oxethylierte Fettalkoholsulfate, Alkylsulfonate, Alkylbenzolsulfonate, Alkyltrimethylammoniumsalze, Alkylbetaine, oxethylierte Fettalkohole, oxethylierte Nonylphenole, Fettsäurealkanolamide, oxethylierte Fettsäureester, ferner Verdicker wie zum Beispiel höhere Fettalkohole, Stärke, Cellulosederivate, Vaseline, Paraffinöl und Fettsäuren sowie Pflegestoffe wie zum Beispiel kationische Harze, Lanolinderivate, Cholesterin, Pantothensäure und Betain.

Die erwähnten Bestandteile werden in den für solche Zwecke üblichen Mengen verwendet, zum Beispiel die Netzmittel und Emulgatoren in Konzentrationen von etwa 0,5 bis 30 Gewichtsprozent, die Verdicker in einer Menge von etwa 0,1 bis 25 Gewichtsprozent und die Pflegestoffe in einer Konzentration von etwa 0,1 bis 5,0 Gewichtsprozent. Je nach Zusammensetzung können die erfindungsgemäßen Haarfärbemittel schwach sauer, neutral oder alkalisch reagieren. Insbesondere weisen sie einen pH-Wert zwischen 6,8 und 11,5 auf, wobei der alkalische Bereich zwischen pH 8,0 und 11,0 besonders bevorzugt ist und die Einstellung vorzugsweise mit Ammoniak erfolgt. Es können aber auch organische Amine, zum Beispiel Monoethanolamin und Triethanolamin oder auch anorganische Basen wie zum Beispiel Natriumhydroxid und Kaliumhydroxid Verwendung finden.

Für die Anwendung zur oxidativen Färbung von Haaren vermischt man das vorstehend beschriebene Haarfärbemittel unmittelbar vor dem Gebrauch mit einem Oxidationsmittel und trägt eine für die Haarfärbebehandlung ausreichende Menge, je nach Haarfülle, im allgemeinen etwa 60 bis 200 g, dieses Gemisches auf das Haar auf.

Als Oxidationsmittel zur Entwicklung der Haarfärbung kommen hauptsächlich Wasserstoffperoxid, beispielsweise als 6prozentige wäßrige Lösung, dessen Additionsverbindungen an Harnstoff, Melamin, Natriumborat oder Natriumcarbonat in Form der 3 bis 12prozentigen, vorzugsweise 6prozentigen, wäßrigen Lösungen, aber auch Luftsauerstoff, in Betracht. Wird eine 6prozentige Wasserstoffperoxidlösung als Oxidationsmittel verwendet, so beträgt das Gewichtsverhältnis zwischen Haarfärbemittel und Oxidationsmittel 5:1 bis 1:2, vorzugsweise jedoch 1:1. Größere Mengen an Oxidationsmittel werden vor allem bei höheren Farbstoffkonzentrationen im Haarfärbemittel oder wenn gleichzeitig eine stärkere Bleichung des Haares beabsichtigt ist, verwendet. Man läßt das Gemisch bei 15 bis 50 Grad Celsius etwa 10 bis 45 Minuten lang, vorzugsweise 30 Minuten lang, auf das Haar einwirken, spült sodann das Haar mit Wasser aus und trocknet es. Gegebenenfalls wird im Anschluß an die Spülung mit einem Shampoo gewaschen und eventuell mit einer schwachen organischen Säure wie zum Beispiel Zitronensäure oder Weinsäure nachgespült. Anschließend wird das Haar getrocknet. Die Entwicklersubstanzen sollen in dem Haarfärbemittel entweder als freie Base oder in Form seiner physiologisch verträglichen wasserlöslichen Salze mit anorganischen oder organischen Säuren, zum Beispiel als Chlorid, Sulfat, Phosphat, Acetat, Propionat, Lactat oder Citrat eingesetzt werden.

Die Entwicklersubstanzen sind gut in Wasser löslich, sie weisen außerdem eine ausgezeichnete Lagerstabilität, insbesondere als Bestandteil des hier beschriebenen Haarfärbemittels auf. Das erfindungsgemäße Haarfärbemittel auf der Basis von 2-(2,5-Diaminophenyl)ethanol und 4-Amino-2-aminomethyl-phenol als Entwicklersubstanzen ermöglicht Haarfärbungen mit ausgezeichneten Echtheitseigenschaften, insbesondere was die Licht-, Wasch- und Reibechtheit anbetrifft und läßt sich mit einem Reduktionsmittel wieder abziehen. Hinsichtlich der färberischen Möglichkeiten bietet das erfindungsgemäße Haarfärbemittel, je nach Art und Zusammensetzung der Farbkomponenten, eine breite Palette verschiedener Farbnuancen, welche sich von blonden über braune, purpurne, violette bis hin zu blauen und schwarzen Farbtönen erstreckt. Hierbei zeichnen sich die Farbtöne durch ihre besondere Farbintensität aus.

Die sehr guten färberischen Eigenschaften des Haarfärbemittels gemäß der vorliegenden Anmeldung zeigen sich weiterhin darin, daß dieses Mittel eine Anfärbung von ergrauten, chemisch nicht vorgeschädigten Haaren problemlos und mit guter Deckkraft ermöglicht.

In dem nachstehenden Beispiel soll der Gegenstand der Erfindung näher erläutert werden, ohne ihn auf das Beispiel zu beschränken.

**Beispiel für Haarfärbemittel**

**Beispiel 1: Haarfärbemittel in Cremeform**

| | |
|---|---|
| 0,34 g | 2-(2,5-Diaminophenyl)ethanol-sulfat |
| 1,31 g | 4-Amino-2-aminomethyl-phenol-hydrochlorid |
| 0,24 g | Resorcin |
| 0,30 g | m-Aminophenol |
| 0,24 g | 2-Amino-4-(2'-hydroxyethyl)aminoanisolsulfat |
| 0,20 g | 5-Amino-2-methyl-phenol |
| 0,05 g | 1-[(2'-Ureidoethyl)amino]-4-nitrobenzol |
| 15,00 g | Cetylalkohol |
| 3,50 g | Natrium-Laurylalkohol-diglykolethersulfat (28prozentige wäßrige Lösung) |
| 3,00 g | Ammoniak(25prozentige wäßrige Lösung) |
| 0,30 g | Natriumsulfit (wasserfrei) |
| 75,52 g | Wasser |
| 100,00 g | |

10 g dieses Haarfärbemittels werden kurz vor Gebrauch mit 10 ml Wasserstoffperoxidlösung (6prozentig) vermischt. Anschließend trägt man das Gemisch auf blonde Naturhaare auf und läßt es 30 Minuten lang bei 40 Grad Celsius einwirken. Danach wird das Haar mit Wasser gespült und getrocknet. Das Haar hat eine palisanderfarbene Färbung erhalten.

Alle Prozentangaben stellen, soweit nicht anders angegeben, Gewichtsprozente dar.

**Patentansprüche**

1. Mittel zur oxidativen Färbung von Haaren mit einem Gehalt an einer Entwicklersubstanz-Kupplersubstanz-Kombination, dadurch gekennzeichnet, daß es als Entwicklersubstanz eine Kombination aus 2-(2,5-Diaminophenyl)ethanol und 4-Amino-2-aminomethyl-phenol enthält.

2. Mittel nach Anspruch 1, dadurch gekennzeichnet, daß es die Entwicklersubstanz in einer Menge von 0,01 bis 5,0 Gewichtsprozent enthält.

3. Mittel nach Anspruch 1 oder 2, dadurch gekennzeichnet, daß die Entwicklersubstanz-Kupplersubstanz-Kombination in einer Menge von 0,02 bis 8,0 Gewichtsprozent enthalten ist.

4. Mittel nach einem der Ansprüche 1 bis 3, dadurch gekennzeichnet, daß es einen pH-Wert von 6,8 bis 11,5 aufweist.

EP 0 778 019 A1

**EUROPÄISCHER RECHERCHENBERICHT**

Nummer der Anmeldung

EP 96 11 6147

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
|---|---|---|---|
| X | WO 87 03475 A (WELLA AG) 18.Juni 1987<br>* Seite 2, Zeile 13 - Seite 3, Zeile 20 *<br>* Seite 4, Zeile 7 - Zeile 11; Anspruch 8 * | 1-4 | A61K7/13 |
| P,X | EP 0 722 710 A (L'OREAL) 24.Juli 1996<br>* Ansprüche 1,4,5,10,13 * | 1,2,4 | |
| A | EP 0 667 142 A (L'OREAL) 16.August 1995<br>* Anspruch 1 * | 1 | |
| A | DE 43 14 317 A (HENKEL KGAA) 3.November 1994<br>* Seite 9, Zeile 25 *<br>* Seite 9, Zeile 30 *<br>* Seite 2, Zeile 43 - Seite 3, Zeile 42 * | 1 | |
| A | DE 43 17 855 A (HENKEL KGAA) 1.Dezember 1994<br>* Seite 6, Zeile 5 *<br>* Seite 6, Zeile 17 *<br>* Seite 2, Zeile 29 - Seite 3, Zeile 6 * | 1 | |
| A | EP 0 007 537 A (WELLA AG) 6.Februar 1980<br>* Ansprüche 1,3 * | 1 | RECHERCHIERTE SACHGEBIETE (Int.Cl.6)<br><br>A61K |
| A | WO 80 01241 A (WELLA AG ;HUSEMEYER H (DE); KONRAD E (DE); MAGER H (DE)) 26.Juni 1980<br>* Ansprüche 1,4 * | 1 | |

Der vorliegende Recherchenbericht wurde für alle Patentansprüche erstellt

| Recherchenort | Abschlußdatum der Recherche | Prüfer |
|---|---|---|
| BERLIN | 18.März 1997 | McConnell, C |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
...............................................
& : Mitglied der gleichen Patentfamilie, übereinstimmendes Dokument

EPO FORM 1503 03.82 (P04C03)